(19) Europäisches Patentamt
European Patent Office
Office européen des brevets

(11) Publication number: **0 430 621 A2**

(12) **EUROPEAN PATENT APPLICATION**

(21) Application number: **90312831.2**

(22) Date of filing: **26.11.90**

(51) Int. Cl.5: **C07D 301/26**, C07D 301/32, A61K 31/335, A61K 31/35, C07D 493/04, //(C07D493/04, 311:00,303:00)

The title of the invention has been amended (Guidelines for Examination in the EPO, A-III, 7.3).

(30) Priority: **29.11.89 GB 8926993**
**25.10.90 GB 9023300**

(43) Date of publication of application:
**05.06.91 Bulletin 91/23**

(84) Designated Contracting States:
**AT BE CH DE DK ES FR GB GR IT LI LU NL SE**
**Bulletin**

(71) Applicant: **Beecham Group p.l.c.**
**SB House Great West Road**
**Brentford Middlesex TW8 9BD(GB)**

(72) Inventor: **Smith, Paul, SmithKline Beecham Pharmaceuticals**
**Coldharbour Road, The Pinnacles**
**Harlow, Essex CM19 5AD(GB)**
Inventor: **Ghavshou, Michael, SmithKline Beecham**
**Pharmaceuticals, Coldharbour Road, The Pinnacles**
**Harlow, Essex CM19 5AD(GB)**

(74) Representative: **Jones, Pauline et al**
**SmithKline Beecham, Corporate Patents,**
**Great Burgh, Yew Tree Bottom Road**
**Epsom, Surrey KT18 5XQ(GB)**

(54) **Process for the preparation of chiral 3,4-epoxy benzopyrans.**

(57) A process for the preparation of a compound of formula (A)' or (A)":

EP 0 430 621 A2

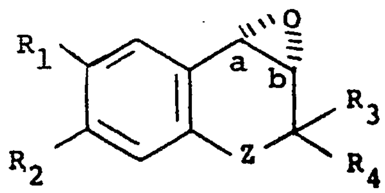

(A)'

(S,S)-isomer

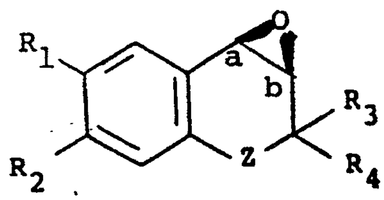

(A)''

(R,R)-isomer

which process comprises the base treatment of a quaternary ammonium salt of a compound of formula (D):

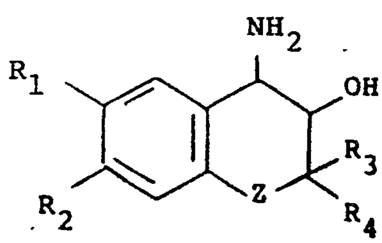

(D)

wherein the $NH_2$ and OH moieties are trans and are (R,S) or (S,R), respectively.

## PROCESS

The present invention relates to a novel process for the preparation of chiral 3,4-epoxy benzopyrans and corresponding compounds wherein the pyran oxygen is replaced by another moiety, or a bond, to form tetrahydronaphthalene and indane derivatives, respectively.

EP-A-76075, 91748, 95316, 107423, 120426, 126367, 126311, 168619, 172352, 205292, 214818, 250077, 321175, 322251 and 376524 (Beecham Group p.l.c.) describe classes of compounds which are potassium channel activators which relax smooth muscle, and are therefore useful as inter alia antihypertensive agents and bronchodilators.

EP-A-273262 and 363883 (Merck Patent GmbH), EP-A-277611 and 277612 (Hoechst Aktiengesellschaft), EP-A-314446 (American Home Products Corporation), EP-A-296975 (Sanofi) and WO 89/07103 (Nissan Chemical Industries Ltd.) describe further classes of benzopyran derivatives which are believed to be potassium channel activators.

Potassium channel activators are also of potential use in the treatment of irritable bowel syndrome, diverticular disease, premature labour, incontinence, congestive heart failure, angina, peripheral vascular disease, cerebral vascular disease, pulmonary hypertension and renal cholic. They may also be of potential use in the treatment of epilepsy and glaucoma.

EP-A-120428 (Beecham Group p.l.c.) describes benzopyrans which are (3S,4R)-isomers of some of the compounds described in the aforementioned European Patent publications in the name of Beecham Group p.l.c. EP-A-277611 describes the (3R,4S)-isomers of the compounds described therein.

The synthesis of the aforementioned compounds is achieved from an epoxide intermediate of formula (A):

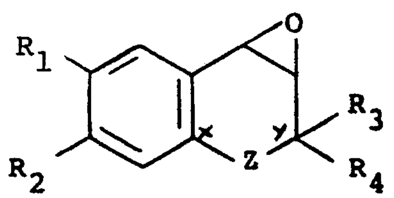

(A)

wherein

Z is oxygen, $CH_2$ or z is a bond;

one of $R_1$ and $R_2$ is hydrogen or a substituent and the other is a substituent, such as defined in the aforementioned patent publications; and

$R_3$ and $R_4$ are independently hydrogen or $C_{1-6}$ alkyl or together are $C_{2-5}$ polymethylene.

It will be appreciated that, when Z is a bond, the compound of formula (A) is an epoxyindane derivative, i.e. such that the carbon atoms marked x and y in formula (A) are joined directly together.

Preferably $R_1$ is a substituent and $R_2$ is hydrogen. $R_1$ is preferably nitro, cyano, acetyl, $CF_3$, $C_2F_5$, $OCF_3$, $C_{1-6}$ alkyl, such as methyl, ethyl or isopropyl, or $C_{3-8}$ cycloalkyl, such as cylopentyl.

$R_3$ and $R_4$ are preferably both methyl groups when Z is oxygen or $CH_2$.

The general process for preparing trans-4-amino-3-hydroxy benzopyrans and corresponding compounds wherein Z is other than oxygen, involves ring opening the epoxide intermediate with a nucleophile to form a trans 4-substituted-3-hydroxy

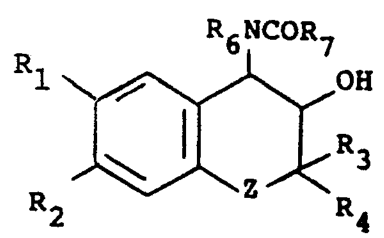

(I)

wherein
Z and $R_1$ to $R_4$ are as defined in formula (A); $R_6$ and $R_7$ are as defined for the corresponding variables in the aforementioned patent publications; and the $R_6NCOR_7$ and OH moieties are trans.

The epoxide intermediates of formula (A) are generally prepared from the corresponding compound of formula (B), which is a chromene when Y is C-$R_1$ and Z is oxygen:

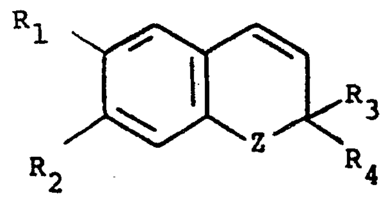

(B)

Epoxidation is generally achieved by conventional synthetic methods, such as conversion of the compound of formula (B) to the corresponding bromohydrin of formula (C):

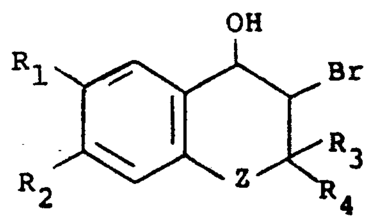

(C)

wherein the Br and OH moieties are trans, by reaction with HOBr , followed by treatment with sodium hydride.

A novel process has now been discovered which resolves a compound of formula (A) to a compound of formula (A) which is enantiomerically pure, which is a useful intermediate in the preparation of chiral compounds of formula (I).

Accordingly, the present invention provides a process for the preparation of a compound of formula (A) in which the configuration at the carbon atoms a and b are both R or both S i.e. (A' or A"):

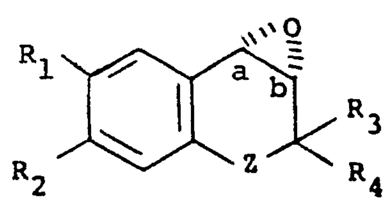

(A)'

(S,S)-isomer

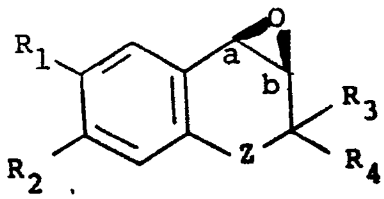

(A)''

(R,R)-isomer

which process comprises the base treatment of a quaternary ammonium salt of a compound of formula (D):

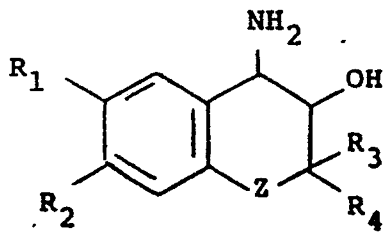

(D)

wherein the $NH_2$ and OH moieties are trans and are (R,S) or (S,R), respectively.

Suitable quaternary ammonium salts include those formed with RL wherein R is $C_{1-4}$ alkyl and L is halogen or sulphate. Preferably R is methyl and L is iodo. The quaternary salt is then formed by reaction with RL in a suitable solvent, such as acetonitrile or dimethylformamide, or alcohols, such as methanol, in the presence of a base, such as sodium or potassium carbonate, at ambient temperature.

The quaternary ammonium salt may be treated with a suitable base, such as sodium or potassium alkoxides, such as tert-butoxide in a suitable solvent, such as tetrahydrofuran or alcohols, such as $C_{1-4}$ alkanols, at a temperature of 0-50°C. Silver oxide may also be utilised as the 'base'.

Compounds of the formula (D) in racemic form may be prepared as described in the aforementioned patent publications, usually by treatment of a compound of formula (A) with ammonium hydroxide.

The desired isomer of the compound of formula (D) is preferably obtained by fractional crystallization of a suitable derivative. A suitable resolving agent is (+)- or (-)-endo-3-bromocamphor-9-sulphonic acid as the ammonium salt. Other camphor sulphonic acids may also be used, or acids such as tartaric acids, substituted tartaric acids, mandelic acids, such as (+)-mandelic acid, and nitrotartranilic acids. Suitable solvents are lower (e.g. $C_{1-5}$) alcohols such as ethanol or isopropyl alcohol, possibly with added water. With some acids, polar organic solvents such as esters and ketones may be suitable.

A particularly preferred compound which can be prepared from the compound of formula (A)' is (-)-trans-6-cyano-3,4-dihydro-2,2-dimethyl-4-(2-oxo-1-pyrrolidinyl)-2H-1-benzopyran-3-ol, known as BRL 38227, obtainable by reacting pyrrolidone anion with (3S,4S)-6-cyano-3,4-dihydro-3,4-epoxy-2,2-dimethyl-2H-1-benzopyran.

The following illustrate the invention, and the following Description relates to the preparation of the compound of formula (D).

Description

Resolution of (±)-trans-4-Amino-6-cyano-3,4-dihydro-2,2-dimethyl-2H-1-benzopyran-3-ol

The title compound (100g) was dissolved in isopropyl alcohol (500ml) with stirring and heating to 70°C. Water (250ml) was added followed by (+)-ammonium 3-bromo-camphor-9-sulphonate (150.5g). The mixture was stirred and warmed back to 70°C to effect dissolution. 5N Hydrochloric acid (80ml) was then added fairly rapidly until the mixture reached pH5. It was then cooled to 55°C before seeding with authentic crystalline product. The mixture was cooled to room temperature before filtering off the product and washing with a mixture of isopropyl alcohol (50ml) and water (25ml). After drying in air at 50°C the yield of 3-bromo-camphor-9-sulphonic acid salt of the (+)-isomer of the title compound was 75g (31%).

$[\alpha]_D^{20}$ (c=1, MeOH ) = + 88.9°, m.p. 288-291°C. δ($d_6$-DMSO): 0.81 (s, 3H); 1.07 (s, 3H); 1.15 (s, 3H); 1.10-1.25 (m, 1H); 1.45 (s, 3H); 1.66-1.88 (m, 2H); 2.05-2.20 (m, 1H), 2.36 (d, J = 14Hz, 1H); 2.83 (d, J = 14Hz, 1H); 2.97 (ss, J = 6, 6Hz, 1H); 3.64 (dd, J = 6, 10Hz, 1H); 4.30 (d, J = 10Hz, 1H); 5.00 (d, J = 6Hz, 1H); 6.42 (d, J = 6Hz, 1H); 7.04 (d, J = 8Hz, 1H); 7.76 (m, 1H); 8.07 (bs, 1H) ; 8.53 (bs, 3H).

Having thus obtained the (3S,4R)-isomer as its (+)-3-bromo-camphor-9-sulphonic acid salt the residual solution was treated with aqueous sodium hydroxide until basic and was extracted with dichloromethane. Evaporation gave a crude product with the approximate composition 80-85% (3R,4S)-isomer and 15-20% (3S,4R)-isomer.

This crude product (250g) was triturated with diisopropyl ether (1200ml) and concentrated hydrochloric acid (100ml) to crystallise the hydrochloride salt. The mixture was cooled to room temperature before filtering off the product and washing with diisopropyl ether. After drying in air at 50°C the yield of the hydrochloride salt was 267g (91%), the isomer ratio being unchanged from that of the crude product.

The hydrochloride salt (127.25g) together with (+)-tartaric acid (75.0g) were dissolved in propan-2-ol (200ml) and water (400ml). To this solution was added a solution of sodium hydroxide (20g) in water (100ml) and the mixture was allowed to stand for several days. During this time a tartrate salt crystallised that was shown to have the approximate composition 50% (3R,4S)-isomer and 50% (3S,4R)-isomer. As this salt crystallised and was filtered off the proportion of (3R,4S)-isomer in the mother liquors increased to ca. 98% at which time no further tartrate salt crystallised.

The solution was basified by the addition of sodium hydroxide (30g) in water (100ml) and extracted with dichloromethane (2 x 600ml). Evaporation afforded the (3R,4S)-isomer as a glassy solid which was dissolved in propan-2-ol (200ml) with heating. Concentrated hydrochloric acid (60ml) was added to the solution which was cooled to 0°C to crystallise the compound. This was collected by filtration, washed with cold propan-2-ol (100ml) and dried in air at 60°C. Evaporation of the propan-2-ol liquors to ca. 100ml and cooling to 0°C afforded a second crop of the compound as its HCl salt. Again this was collected by filtration, washed with cold propan-2-ol (50ml) and dried in air at 60°C.

These two crops afforded 67.2g (57%) of the (3R,4S)-isomer.

$[\alpha]_D^{20}$ (c=1, MeOH ) = -63.04°

m.pt:- 262-265°C

δ ($d_6$-DMSO) : 1.14 (s, 3H); 1.44 (s, 3H); 3.66 (dd, J = 5.6, 9.5Hz, 1H); 4.25 (d, J = 9.5Hz, 1H), 6.45 (d, J = 5.6Hz, 1H); 7.01 (d, J=8.7Hz, 1H); 7.73 (dd, J = 2.0, 8.7Hz, 1H); 8.21 (bs, 1H); 8.76 (bs, 3H).

Example 1

a) (3S,4R)-6-Cyano-3,4-dihydro-2-2-dimethyl-3-hydroxy-4-trimethylammonium-2H-1-benzopyran iodide

(3S,4R)-4-amino-6-cyano-3,4-dihydro-2,3-dimethyl-1H-1-benzopyran-3-ol (+)-3-bromocamphor-9-sulphonate (100g) was suspended in water (1.5L) containing methylene chloride (200ml) and treated with aqueous sodium hydroxide solution (85ml, 10%). The organic phase was separated and the aqueous solution was extracted with two further portions of methylene chloride (100ml). The combined organic extracts were dried with sodium sulphate, filtered and the solvent removed to leave an oily residue.

The residue obtained above was dissolved in dimethylformamide (750ml) and sodium carbonate (82.2g) was added followed by methyl iodide (50ml). An ice bath was applied until the initial, slightly exothermic, reaction had subsided, after which a second portion of methyl iodide (50ml) was added and the reaction was allowed to proceed at room temperature for 96 hours.

The solvent was removed and the residue suspended in water (2.0L). Sodium chloride was added until a saturated solution was obtained. The product was then filtered and dried, initially in air and finally under high vacuum to give a white solid.

| | |
|---|---|
| Yield: | 58.6g |
| m.pt: | 186 - 189°C (dec) |
| $[\alpha]D^{20}$: | +69.57° c = 1 in water |

δ ( $CD_3OD$ ) : 0.98 (s, 3H); 1.62 (s, 3H); 3.19 (s, 9H) 4.52 (d, J = 4.4Hz, 1H); 4.76 (d, J = 4.4Hz, 1H), 7.20 (d, J = 8.4Hz, 1H); 7.85 (dd, J=8.4Hz, 2.0Hz, 1H); 8.06 (d, J = 2.0Hz, 1H).

b) (3S,4S)-6-Cyano-3,4-dihydro-3,4-epoxy-2,2-dimethyl-2H-1-benzopyran

(3S,4R)-6-cyano-3,4-dihydro-2,2-dimethyl-3-hydroxy-4-trimethylammonium-2H-1-benzopyran iodide (56.8g) was suspended in dry tetrahydrofuran (500ml), cooled to 0-5°C and treated dropwise with a solution of potassium tert. butoxide (17.95g) in tetrahydrofuran (250ml) over about fifteen minutes.

After one hour the solvent was removed and the residue suspended in methylene chloride (400ml) and

washed with dilute sodium bicarbonate solution (1 x 200ml and 2 x 100ml). The organic solution was dried with sodium sulphate, filtered and evaporated to give the product as a white solid which was recrystallized from aqueous propan-2-ol.

Yield: 25.5g
m.pt: 141 - 142°C
$[\alpha]_D^{20}$ : -87.97° c = 1.2 in $CH_2Cl_2$

δ ( $CDCl_3$) : 1.31 (s, 3H) ; 1.61 (s, 3H) ; 3.56 (d, J = 4.3Hz, 1H); 3.92 (d, J = 4.3Hz, 1H), 6.87 (d, J = 8.4Hz, 1H); 7.54 (dd, J=8.4Hz, 2.0Hz, 1H); 7.66 (d, J = 2.0Hz, 1H).

Example 2

a) (3R,4S)-6-Cyano-3,4-dihydro-2,2-dimethyl-3-hydroxy-4-trimethylammonium-2H-1-benzopyran iodide

(3R,4S)-4-amino-6-cyano-3,4-dihydro-2,2-dimethyl-1H-benzopyran-3-ol hydrochloride (2.54g) was dissolved in dimethylformamide (50ml) and sodium carbonate (4.24g) was added, followed by methyl iodide (2.5ml). An ice bath was applied until the initial, slightly exothermic, reaction had subsided, after which a second portion of methyl iodide (2.5ml) was added and the reaction was allowed to proceed at room temperature for 96 hours.

The solvent was removed and the residue suspended in water (150ml). Sodium chloride was added until a saturated solution was obtained. The product was filtered and dried, initially in air and finally under high vacuum to give a white solid.

Yield: 3.02g
m.pt: 186 - 189°C (dec)
$[\alpha]_D^{20}$: -64.21° c = 1 in water

δ ( $CD_3OD$ ) : 0.98 (s, 3H); 1.62 (s, 3H); 3.19 (s, 9H); 4.52 (d, J = 4.4Hz, 1H); 4.76 (d, J = 4.4Hz, 1H), 7.20 (d, J = 8.4Hz, 1H); 7.85 (dd, J=8.4Hz, 2.0Hz, 1H); 8.06 (d, J = 2.0Hz, 1H).

b) (3R, 4R)-6-Cyano-3, 4-dihydro-3, 4-epoxy-2, 2-dimethyl-2H-1-benzopyran

(3R,4S)-6-cyano-3,4-dihydro-2,2-dimethyl-3-hydroxy-4-trimethylammonium-2H-1-benzopyran iodide (2.84g) was suspended in dry tetrahydrofuran (50ml), cooled to 0-5°C and treated dropwise with a solution of potassium tert. butoxide (0.902g) in tetrahydrofuran (25ml) over about five minutes.

After one hour the solvent was removed and the residue suspended in methylene chloride (50ml) and washed with dilute sodium bicarbonate solution (1 x 40ml, 2 x 20ml). The organic solution was dried with sodium sulphate, filtered and evaporated to give the product as a white solid, which was recrystallized from aqueous propan-2-ol.

Yield: 1.169g
m.pt: 141 - 142°C
$[\alpha]_D^{20}$: +89.56° c = 1.2 in $CH_2Cl_2$

δ ( $CDCL_3$) : 1.31 (s, 3H); 1.61 (s, 3H) ; 3.56 (d, J = 4.3Hz, 1H); 3.92 (d, J = 4.3Hz, 1H), 6.87 (d, J = 8.4Hz, 1H); 7.54 (dd, J=8.4Hz, 2.0Hz, 1H); 7.66 (d, J = 2.0Hz, 1H).

Example 3

a) (3S, 4R)-6-Ethyl-4-amino-3, 4-dihydro-2, 2-dimethyl-2H-1-benzopyran-3-ol (+)-mandelate

(+)-Mandelic acid (0.57 kg) and trans-6-ethyl-4-amino-3,4-dihydro-2,2-dimethyl-2H-1-benzopyran-3-ol **(0.75kg) were dissolved in warm 2-propanol (18 L). The solution was concentrated to 12 L and crystallised to give the title compound (0.523 kg). $[\alpha]_D^{20}$ = +56.5°, c = 1 in MeOH .

b) (3S 4R)-6-Ethyl-3, 4-dihydro-2, 2-dimethyl-3-hydroxy-4-trimethylammonium-2H-1-benzopyran iodide

(3S,4R)-6-Ethyl-4-amino-3,4-dihydro-2,2-dimethyl-2H-1-benzopyran-3-ol (+)-mandelate (2.0 g) was dissolved in methylene chloride (150 mL) and sodium hydroxide solution (50 mL, 5%) added. The mixture was stirred vigorously for 1 h, then separated. The organic layer was dried and evaporated to give a colourless solid which was dissolved in DMF (20 mL) at 0°C and sodium carbonate (2.23 g) added. Methyl iodide (1.3 mL) was then added and the reaction mixture stirred at room temperature for 3 h, after which time a further 1.3 mL of methyl iodide was added. The reaction mixture was stirred at room temperature for 96 h, then the

** EP-A-250077 (Beecham Group p.l.c.).

solvents were removed in vacuo, and water (50 mL) added. The product was filtered to give the title compound as a solid (1.49 g).

c) (3S,4S)-6-Ethyl-3,4-dihydro-3,4-epoxy-2,2-dimethyl-2H-1-benzopyran

(3S,4R)-6-Ethyl-3,4-dihydro-2,2-dimethyl-3-hydroxy-4-trimethylammonium-2H-1-benzopyran iodide (1.49 g) was suspended in dry THF (20 mL) and a suspension of potassium tert-butoxide (0.47 g) in dry THF (10 mL) added dropwise at 0° C. The reaction mixture was stirred at room temperature for 3 h, then the solvent removed in vacuo and the residue partitioned between methylene chloride and dilute sodium bicarbonate solution. The organic layer was washed with sodium bicarbonate solution, brine and then dried. Evaporation of solvent in vacuo gave the title compound as a solid (0.7 g).
$[\alpha]_D^{20}$ = -13.71°, c = 0.992 in $CHCl_3$.

## Claims

1. A process for the preparation of a compound of formula (A)' or (A)":

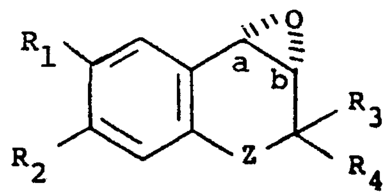

(A)'

(S,S)-isomer

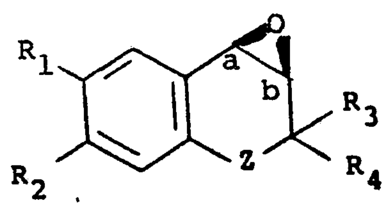

(A)''

(R,R)-isomer

which process comprises the base treatment of a quaternary ammonium salt of a compound of formula (D):

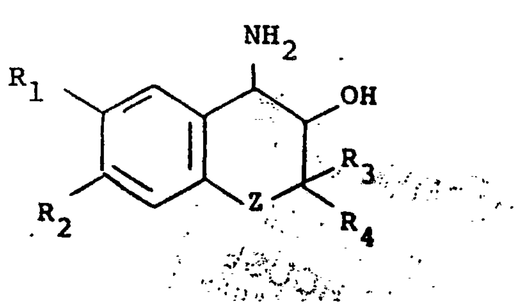

(D)

wherein the $NH_2$ and OH moieties are trans and are (R,S) or (S,R), respectively.

2. A process according to claim 1 wherein Z is oxygen.

3. A process according to claim 1 or 2 wherein $R_1$ is a substituent and $R_2$ is hydrogen.

4. A process according to claim 1, 2 or 3 wherein $R_3$ and $R_4$ are both methyl groups.

5. A process according to any one of claims 1 to 4 wherein $R_1$ is nitro, cyano, acetyl, $CF_3$, $C_2F_5$, $OCF_3$, ethyl, isopropyl or cyclopentyl.

6. A process according to any one of claims 1 to 5 for the preparation of a compound of formula (A') i.e. the (S,S)-isomer.

7. A process according to claim 1, for the preparation of:
   (35,4S)-6-cyano-3,4-dihydro-3,4-epoxy-2,2-dimethyl-2H-1-benzopyran,
   (3R,4R)-6-cyano-3,4-dihydro-3,4-epoxy-2,2-dimethyl-2H-1-benzopyran, or
   (3S,4S)-6-ethyl-3,4-dihydro-3,4-epoxy-2,2-dimethyl-2H-1-benzopyran.

8. A process according to claim 1 wherein the quaternary ammonium salt is that formed with RL wherein R is $C_{1-4}$alkyl and L is hydrogen.

9. A process according to claim 8, wherein RL is $CH_3I$ .

10. A (3S,4R) or (4R,4S) potassium channel activator benzopyran whenever prepared from a (3S,4S) or (3R,4R) epoxide of formula (A') or (A'') respectively, as prepared according to claim 1.